# EUROPÄISCHE PATENTSCHRIFT

(11) **EP 2 167 978 B1**
(45) Veröffentlichungstag und Bekanntmachung des Hinweises auf die Patenterteilung: **28.10.2015**
(21) Anmeldenummer: 08773827.4
(22) Anmeldetag: 03.07.2008
(51) Int. Cl.: G01N 33/86, C12Q 1/37

(54) **VERFAHREN ZUR BESTIMMUNG DER VON WILLEBRAND FAKTOR-AKTIVITÄT IN ABWESENHEIT VON RISTOCETIN**
METHOD FOR DETERMINING VON WILLEBRAND FACTOR ACTIVITY IN THE ABSENCE OF RISTOCETIN
PROCÉDÉ POUR DÉTERMINER L'ACTIVITÉ DU FACTEUR VON WILLEBRAND EN L'ABSENCE DE RISTOCÉTINE

(30) Priorität: 06.07.2007 DE 102007031708
(43) Veröffentlichungstag der Anmeldung: 31.03.2010
(60) Teilanmeldung: 11002342.1
(73) Patentinhaber: Siemens Healthcare Diagnostics Products GmbH, 35041 Marburg (DE)
(72) Erfinder: ALTHAUS, Harald, 35083 Wetter (DE); OBSER, Tobias, 22455 Hamburg (DE); PATZKE, Juergen, 35043 Marburg (DE); SCHNEPPENHEIM, Reinhard, 22399 Hamburg (DE)
(86) Internationale Anmeldenummer: PCT/EP2008/005416
(87) Internationale Veröffentlichungsnummer: WO 2009/007051

(56) Entgegenhaltungen:
- AU-A1- 2006 241 373
- US-A1- 2005 186 646
- BISWAS TAPAN K ET AL: "Properties of soluble his-tagged rGPIb alpha(1-483) wild-type and increase-of-function mutants." BLOOD, Bd. 106, Nr. 11, Part 2, November 2005 (2005-11), Seite 67B, XP002512023 & 47TH ANNUAL MEETING OF THE AMERICAN-SOCIETY-OF-HEMATOLOGY; ATLANTA, GA, USA; DECEMBER 10 -13, 2005 ISSN: 0006-4971
- TAIT A SASHA ET AL: "Phenotype changes resulting in high-affinity binding of von Willebrand factor to recombinant glycoprotein Ib-IX: Analysis of the platelet-type von Willebrand disease mutations" BLOOD, Bd. 98, Nr. 6, 15. September 2001 (2001-09-15), Seiten 1812-1818, XP002512024 ISSN: 0006-4971
- FEDERICI A B ET AL: "A sensitive ristocetin co-factor activity assay with recombinant glycoprotein Ib[alpha] for the diagnosis of patients with low von Willebrand factor levels" HAEMATOLOGICA, FONDAZIONE FERRATA STORTI, ROME, IT, Bd. 89, Nr. 1, 1. Januar 2004 (2004-01-01), Seiten 77-85, XP002390136 ISSN: 0390-6078
- VANHOORELBEKE KAREN ET AL: "A reliable and reproducible ELISA method to measure ristocetin cofactor activity of von Willebrand factor" THROMBOSIS AND HAEMOSTASIS, STUTTGART, DE, Bd. 83, Nr. 1, 1. Januar 2000 (2000-01-01), Seiten 107-113, XP000974940 ISSN: 0340-6245
- HASSENPFLUG WOLF ACHIM ET AL: "Impact of mutations in the von Willebrand factor A2 domain on ADAMTS13-dependent proteolysis." BLOOD 15 MAR 2006, Bd. 107, Nr. 6, 15. März 2006 (2006-03-15), Seiten 2339-2345, XP002520836 ISSN: 0006-4971
- RAYES J ET AL: "Effect of von Willebrand disease type 2B and type 2M mutations on the susceptibility of von Willebrand factor to ADAMTS-13." JOURNAL OF THROMBOSIS AND HAEMOSTASIS : JTH FEB 2007, Bd. 5, Nr. 2, Februar 2007 (2007-02), Seiten 321-328, XP002520837 ISSN: 1538-7933
- KOSTOUSOV ET AL: "Novel, semi-automated, 60-min-assay to determine von Willebrand factor cleaving activity of ADAMTS-13" THROMBOSIS RESEARCH, TARRYTOWN, NY, US, Bd. 118, Nr. 6, 30. Oktober 2006 (2006-10-30), Seiten 723-731, XP005841257 ISSN: 0049-3848
- TRIPODI A ET AL: "Measurement of von Willebrand factor cleaving protease (ADAMTS-13): results of an international collaborative study involving 11 methods testing the same set of coded plasmas." JOURNAL OF THROMBOSIS AND HAEMOSTASIS : JTH SEP 2004, Bd. 2, Nr. 9, September 2004 (2004-09), Seiten 1601-1609, XP002520838 ISSN: 1538-7933

## Beschreibung

Die Erfindung liegt auf dem Gebiet der Gerinnungsdiagnostik und betrifft ein in vitro-Verfahren zur Bestimmung der von Willebrand Faktor (VWF)-Aktivität in einer Probe. Das Verfahren umfasst die Verwendung einer mutanten gain-of-function Variante des GPlbα-Proteins, wodurch auf die Verwendung von Ristocetin, Botrocetin oder einer anderen Ristocetin- oder Botrocetin-äquivalenten Substanz verzichtet werden kann.

Der von Willebrand-Faktor (VWF) ist ein hochmolekulares, multimeres Glykoprotein im Blutplasma, das wichtige Funktionen in dem Prozess der primären Hämostase hat. Der VWF verfügt unter anderem über Bindungsstellen für Kollagen und für das Glykoprotein Ib (GPIb), das auf der Thrombozytenoberfläche lokalisiert ist. GPIB ist ein integrales Membranprotein, das mit einem weiteren integralen Membranprotein, dem Glykoprotein IX (GPIX), den Glykoprotein Ib-IX-Rezeptorkomplex in der Thrombozytenmembran bildet. GPIB ist ein zweikettiges Molekül, das aus einer schweren Kette mit einer apparenten molekularen Masse von etwa 145 kDa (synonym: heavy chain, alpha-Kette oder GPIbα) und einer leichten Kette mit einer apparenten molekularen Masse von etwa 22 kDa (synonym: light chain, beta-Kette oder GPIbα) besteht, welche über Disulfidbrücken miteinander verbunden sind [Lopez, J.A. et al. (1987) Cloning of the α chain of human platelet glycoprotein Ib: A transmembrane protein with homology to leucine-rich α2-glycoprotein. Proc. Natl. Acad. Sci USA 84: 5615-5619].

Im Falle einer Gefäßverletzung werden Kollagenoberflächen exponiert, an die VWF bindet. Infolge der Bindung an Kollagen und unter dem Einfluss der erhöhten Scherkräfte, die auf den Kollagen-gebundenen VWF einwirken, wird VWF so verändert- oder aktiviert, dass er an das aminoterminale Ende der schweren Kette des GPIB (GPIbα) im GPIb-IX-Rezeptorkomplex der Thrombozytenmembran binden kann. Auf diese Weise fängt der aktivierte VWF vorbeiströmende Thrombozyten ein, so dass sich am Ort der Verletzung ein erstes Agglomerat aus VWF, Kollagen und Thrombozyten bildet. In der Folge werden die Thrombozyten aktiviert und damit auch die plasmatische Gerinnung in Gang gesetzt, die letztlich, nach mehreren Verstärkungskaskaden und der Anlagerung weiterer Thrombozyten, zum Wundverschluss führt.

Qualitative oder quantitative Störungen des VWF sind die Ursache eines sogenannten von Willebrand-Syndroms (synonym: von Willebrand Disease, VWD), eines der häufigsten erblichen Blutungsleiden. Zur Diagnose eines von Willebrand-Syndroms stehen verschiedene Screeningverfahren zur Verfügung, wie z. B. die Bestimmung der Blutungszeit (BT), quantitative Verfahren zur Bestimmung der VWF-Antigenkonzentration (VWF:Ag), wie z. B. ELISA-Verfahren, sowie Verfahren zur Bestimmung der VWF-Aktivität, wie z. B. die Ristocetin-induzierte Plättchenagglutination (VWF:RCo).

Das Verfahren der Ristocetin-induzierten Aggregation stabilisierter Thrombozyten, das auch als Ristocetin-Kofaktor-Test bezeichnet wird, erkennt auch solche funktionellen Defekte des VWF-Proteins, die mit quantitativen Verfahren zur Bestimmung der VWF-Antigenkonzentration nicht erkannt werden. Die Durchführung eines Ristocetin-Kofaktor-Tests zur Bestimmung der Ristocetin-Kofaktor-Aktivität ist daher für die komplette Diagnostik eines von Willebrand-Syndroms notwendig. Üblicherweise wird der Ristocetin-Kofaktor-Test durchgeführt, indem eine Plasmaprobe eines Patienten mit fixierten Thrombozyten und mit Ristocetin vermischt wird. Das Ristocetin induziert die Bindung des in der Probe enthaltenen VWF an den GPIb-Rezeptor der zugegebenen Thrombozyten, wodurch die Thrombozyten aggregieren. Das Ausmaß dieser Aggregationsreaktion korreliert mit der Menge an aktivem VWF, das in der Patientenprobe enthalten ist. Die Aggregationsreaktion kann beispielweise durch Messung der Transmissionszunahme optisch erfasst werden und ermöglicht so eine Quantifizierung der VWF:RCo-Aktivität.

Verglichen mit einem VWF-Antigen-Test bietet der Ristocetin-Kofaktor-Test den Vorteil der Aktivitätsbestimmung des VWF und ermöglicht damit die Erkennung funktioneller Störungen des VWF und der Klassifizierung verschiedener Subtypen des von Willebrand-Syndroms, die nur zum Teil auch mit einer verminderten VWF-Antigen-Konzentration einhergehen. Häufig wird zur Subtypen-Klassifizierung das Verhältnis (Ratio) der VWF-Ristocetin-Kofaktor-Aktivität (VWF:RCo) und der VWF-Antigen-Konzentration (VWF:Ag) gebildet. Für die Subtypen 2A, 2B und 2M des von Willebrand-Syndroms ist ein VWF:RCo/VWF:Ag-Ratio kleiner 1 charakteristisch. Als Grenzwert wird häufig ein Ratio von 0,7 empfohlen.

Ein Nachteil des klassischen Ristocetin-Kofaktor-Tests ist die schwierige Automatisierbarkeit des Verfahrens, da die Thrombozyten im Testansatz während der Messung permanent durchrührt werden müssen. Weiterhin nachteilig ist, dass der Test relativ unpräzise ist und die Bestimmung geringer VWF-Aktivitäten im Bereich zwischen 0 und 20 % der Norm nur unzureichend funktioniert.

In jüngerer Zeit wurden GPIbα-basierte VWF-ELISA-Teste entwickelt, bei denen rekombinante GPIbα-Fragmente (1-289 bzw. 1-290) verwendet werden, die die aminoterminale VWF-Bindungsregion enthalten [WO 01/02853 A2 bzw. Vanhoorelbeke, K. et al. (2002) A reliable von Willebrand factor: Ristocetin cofactor enzyme-linked immunosorbent assay to differentiate between type 1 and type 2 von Willebrand disease. Semin Thromb Hemost. 28(2): 161-165 und Federici, A.B. et al. (2004) A sensitive ristocetin co-factor activity assay with recombinant glycoprotein Ibα for the diagnosis of patients with low von Willebrand factor levels. Heamatologica 89(1): 77-85]. Bei diesen Testverfahren wird ein rekombinantes GPIbα-Fragment mit Hilfe eines spezifischen Antikörpers auf einer ELISA-Platte gebunden. Patientenprobe und Ristocetin werden zugegeben, so dass der VWF aus der Patientenprobe an das rekombinante GPIbα-Fragment binden kann. Der gebundene VWF wird schließlich mit Hilfe eines anti-VWF-Antikörpers quantitativ nachgewiesen. Es hat sich gezeigt, dass diese Art der VWF-ELISA-Teste sehr gut mit den Ergebnissen des klassischen Thrombozytenbasierten VWF-Ristocetin-Kofaktor-Aktivitätstest korrelieren und sogar sensitiver und präziser sind.

Hui et al. (Abstract, ISTH 2007) beschreiben einen GPIbα-basierten VWF-ELISA-Test, bei dem rekombinante GPIbα-Fragmente (1-483) mit Mutationen an den Positionen 233 und 239 verwendet werden. Bei diesen Mutationen handelt es sich um sogenannte gain-of-function Mutationen, die in Gegenwart geringer Ristocetinkonzentrationen bekanntermaßen eine höhere Affinität für VWF aufweisen und stärker mit VWF interagieren als wildtypisches GPIbα-Protein (WO 93/16712). Bei den erwähnten Mutationen handelt es sich um seit langem bekannte mutante Varianten der GPIbα-Kette. Die Substitution des Glycin-Restes an Position 233 der GPIbα-Kette durch einen Valin-Rest (G233V) wurde von Miller et al. (US 5,317,097) beschrieben. Diese Mutation ist die Ursache für das Platelet-Type-von-Willebrand-Syndrom (PT-VWD), ein autosomal dominant vererbtes Blutungsleiden. Die Substitution des Methionin-Restes an Position 239 der GPIbα-Kette durch einen Valin-Rest (M239V) wurde von Russell & Roth beschrieben [Russell, S.D. & Roth, G.J. (1993) Pseudo-von Willebrand Disease: A mutation in the platelet glycoprotein Ibα gene associated with a hyperactive surface receptor. Blood 81(7), 1787-1791]. Auch diese Mutation verursacht das PT-VWD.

Ein Nachteil der beschriebenen GPIbα-basierten VWF-ELISA-Teste und auch des Ristocetin-Kofaktor-Tests ist, dass sie auf der Verwendung von Ristocetin bzw. anderen exogenen, nicht-physiologischen Modulatoren beruhen, welche die Bindung von VWF an die GPIbα -Kette vermitteln. Ristocetin, ein antibiotisches Glykopeptid aus dem Bakterium Nocardia lurida, sowie Botrocetin (synonym: Co-Agglutinin), ein Schlangengift aus der Gattung Bothrops, werden bekanntermaßen verwendet, um in vitro die Bindung von VWF an Thrombozyten bzw. an isoliertes GPIbα-Protein oder Fragmente davon zu induzieren. Die Verwendung von Ristocetin hat den Nachteil, dass es nicht nur an VWF, sondern auch an viele andere Proteine, wie z. B. Fibrinogen oder auch anti-GPIbα-Antikörper (beobachtet in eigenen Experimenten) binden kann. Somit besteht bei der Verwendung von Ristocetin in VWF-Testverfahren das Risiko, dass unspezifische Bindungsreaktionen oder Präzipitationsreaktionen auftreten, die das Testergebnis verfälschen können.

Die der Erfindung zugrunde liegende Aufgabe bestand also darin, ein Verfahren zur Bestimmung der VWF-Aktivität bereit zu stellen, das in Abwesenheit von Ristocetin durchgeführt werden kann.

Die Aufgabe wird dadurch gelöst, dass eine gain-of-function Variante der GPIbα-Kette mit zwei Punktmutationen, an Position 233 und an Position 239 bezogen auf die Aminosäuresequenz des humanen GPIbα-Rezeptors, verwendet wird. Unter einer gain-of-function Variante der GPIbα-Kette ist im Sinne der vorliegenden Erfindung eine mutante Variante zu verstehen, die in Gegenwart geringer Ristocetinkonzentrationen bekanntermaßen eine signifikant höhere Affinität für VWF aufweisen und stärker mit VWF interagieren als wildtypisches GPIbα-Protein.

Biswas et al. (BLOOD, Band 106, Nr. 11, Teil 2, Seite 67B) beschreiben ein ELISA-Testsystem, bei dem isoliertes GPIbα-Protein mit einer nicht-partikulären Festphase assoziiert ist und nennen u.a. die Ergebnisse experimenteller Untersuchungen zur Interaktion von vWF mit isoliertem GPIbα-Protein, das an den Positionen 233 und 239 doppelt mutiert ist.

Gegenstand der Erfindung ist ein Verfahren zur Bestimmung der von Willebrand Faktor (VWF)-Aktivität in einer Probe, wobei die Probe mit isoliertem GPIbα-Protein zu einem Testansatz vermischt wird, und dem Testansatz kein Ristocetin und kein Botrocetin hinzugefügt wird. Das verwendete GPIbα-Protein umfasst eine Aminosäuresequenz, die verglichen mit der Wildtypsequenz des humanen GPIbα-Proteins mindestens die Aminosäurereste 1-268 enthält und die an den Positionen 233 und 239 jeweils eine Substitution Xaa aufweist (SEQ ID NO: 1). Das Verwendete GP1bα-Protein ist mit einer partikulären Festphase assoziiert. Bevorzugterweise bestehen die Substitutionen Xaa des Glycin-Rests an Position 233 und des Methionin-Rest an Position 239 der GPIbα-Kette aus einem Valin-Rest (G233V bzw. M239V) oder einem Serin-Rest (G233S bzw. M239S). Jede beliebige Kombination der unterschiedlichen Substitutionen Xaa an den beiden Positionen ist möglich. Bei der Beschreibung der Erfindung ist jede Bezugnahme auf GPIbα-Protein als Bezugnahme auf eine solche mutante Variante zu verstehen.

Bevorzugterweise wird dem Testansatz weiterhin keine "Ristocetin,- oder Botrocetin-äquivalente Substanz" hinzugefügt. Unter dem Begriff "Ristocetin- oder Botrocetin-äquivalente Substanz" ist im Sinne der vorliegenden Erfindung eine exogene, d. h. nicht-physiologische Substanz zu verstehen, die in vitro eine Bindung von gelöstem VWF an die wildtypische GPIbα-Kette oder Fragmente davon zu induzieren vermag.

Ein Vorteil der vorliegenden Erfindung ist, dass durch die Verwendung einer gain-of-function Variante von GPIbα auch auf die Ausübung von Scherstress auf den Testansatz, wie z. B. durch Strömung, Rühren oder Schütteln verzichtet werden kann.

Unter einer "Probe" ist in Bezug auf das Verfahren zur Bestimmung der VWF-Aktivität das Material zu verstehen, das die nachzuweisende Substanz, d. h. den VWF vermutlich enthält. Der Begriff "Probe" umfasst beispielsweise biologische Flüssigkeiten insbesondere von Menschen und Tieren, wie Blut, Plasma oder Serum, sowie industriell gefertigte Produkte, wie z. B. VWF-Kalibratoren oder - Kontrollen oder hochkonzentrierte VWF-Konzentrate, die z. B. für die Substitutionstherapie von von-Willebrand-Syndrom-Patienten vorgesehen sind (z. B. Haemate^{®}P). Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten oder die Zentrifugation von Proben. Der Begriff "Probe" umfasst auch Reaktionsgemische, bestehend aus einem der vorgenannten Probenmaterialien, welchem isolierter VWF als Substrat zugesetzt wurde, um die Aktivität eines VWF-modifizierenden Faktors zu bestimmen, und in denen nach Inkubation des VWF-Substrats mit dem VWF-modifizierenden Faktor die residuale VWF-Aktivität bestimmt werden soll. Beispiele für solche Reaktionsgemische sind beispielweise Gemische aus einer Plasmaprobe mit isoliertem hochmolekularem VWF zur Bestimmung der VWFspaltenden ADAMTS-13 Protease in der Plasmaprobe. Die in der Plasmaprobe enthaltene ADAMTS-13 Protease spaltet das zugesetzte VWF-Substrat und reduziert so die VWF-Aktivität der Probe.

Bei der in dem erfindungsgemäßen Verfahren verwendeten GPIbα-Kette kann es sich um ein rekombinant oder synthetisch hergestelltes GPIbα-Protein handeln. Zur Herstellung von rekombinantem GPIbα-Protein eignen sich bekannte prokaryontische oder eukaryontische Expressionssysteme, wie z. B. die Expression in Bakterien (z. B. E. coli), in Hefen (z. B. Saccharomyces cerevisiae, Pichia pastoris), in pflanzlichen, tierischen oder humanen Zellkulturen. Zur Herstellung von synthetischem GPIbα-Protein eignen sich bekannte Techniken zur in vitro Proteinsynthese, wie z. B. Festphasensynthesen (z. B. Merrifield-Synthese). Bevorzugterweise handelt es sich bei dem in dem erfindungsgemäßen Verfahren verwendeten GPIbα-Protein um rekombinant hergestelltes GPIbα-Protein, das in einer Kultur humaner Zellen, bevorzugt in einer Kultur humaner embryonaler Nierenzellen (HEK-Zellen), hergestellt wurde.

Das in dem erfindungsgemäßen Verfahren verwendete GPIbα-Protein kann am N-Terminus mit der homologen humanen GPIbα-Signalsequenz MPLLLLLLLLPSPLHP (SEQ ID NO: 2, auch als Aminosäurereste -16 bis -1 bezeichnet) fusioniert sein. Alternativ kann das verwendete GPIbα-Protein am N-Terminus mit einer heterologen Signalsequenz fusioniert sein, d. h. mit einem Polypeptid, das üblicherweise nicht in dem humanen GPIbα-Polypeptid vorhanden ist, die aber in dem gewählten Expressionssystem die Expression und/oder Sekretion des rekombinant exprimierten GPIbα-Proteins positiv beeinflusst. Eine geeignete heterologe Signalsequenz ist z. B.
MPLQLLLLLILLGPGNSLQLWDTWADEAEKALGPLLARDRR (SEQ ID NO: 3).

Weiterhin kann das in dem erfindungsgemäßen Verfahren verwendete GPIbα-Protein am C-Terminus mit einem oder mehreren Affinitäts-Tags fusioniert sein, die die Bindung des z. B. rekombinant exprimierten Proteins an einen Affinitätsträger ermöglichen, wodurch z. B. die Reinigung von rekombinant exprimiertem GPIbα-Protein ermöglicht wird oder auch die Bindung des rekombinanten GPIbα-Proteins an eine Festphase. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z. B. spezifische Antikörper, immobilisierte Kationen (z. B. Ni²⁺ mit Affinität für His-Tags) oder andere Typen von Bindungspartnern (z. B. Streptavidin mit Affinität für Strep-Tags).

Im erfindungsgemäßen Verfahren ist das GPIbα-Protein mit einer partikulären Festphase assoziiert. Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist das isolierte GPIbα-Protein über eine chemische Bindung an die Festphase gebunden. Ein Beispiel für eine nicht-kovalente Bindung ist die Oberflächenadsorption. Neben einer direkten Bindung an die Festphase kann das isolierte GPIbα-Protein auch indirekt über spezifische Wechselwirkung mit anderen spezifischen Bindungspartnern an die Festphase gebunden sein, z. B. über spezifische Wechselwirkung mit einem Antikörper, bevorzugt mit einem anti-GPIbα-Antikörper oder -sofern das isolierte GPIbα-Protein über ein Affinitäts-Tag verfügt- mit einem anti-Affinitäts-Tag-Antikörper.

Der Begriff "Partikuläre Festphase" im Sinne dieser Erfindung beinhaltet einen Gegenstand, der aus porösem und/oder nicht porösem, wasserunlöslichem Material besteht und die unterschiedlichsten Partikulären Formen aufweisen kann, wie z. B. Gefäß, Röhrchen, Mikrotitrationsplatte (ELISA-Platte), Kugel, Mikropartikel, etc. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z. B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben.

Die Festphase kann einen Überzug aus einer oder mehreren Schichten aufweisen, z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Eine bevorzugte Ausführungsform des erfindungsgemäßen Verfahrens zur Bestimmung der VWF-Aktivität umfasst die Verwendung eines Partikelassoziierten GPIbα-Proteins, bevorzugt eines Latexpartikel-assoziierten GPIbα-Proteins, und die Bestimmung der VWF-Aktivität anhand der GPIbα-vermittelten Agglutination der partikulären Festphase. Vorzugsweise wird ein über einen Antikörper an die partikuläre Festphase assoziierte GPIbα-Protein verwendet. Zu diesem Zweck sind anti-GPIbα-Antikörper geeignet, insbesondere die monoklonalen Antikörper VM16d [Mazurov, A.V. et al. (1991) Characterization of an antiglycoprotein Ib monoclonals antibody that specifically inhibits plateletthrombin interaction. Thromb Res. 62(6), 673-684; kommerziell erhältlich z. B. von Sanbio B.V., Uden, Niederlande: Produktnummer MON 1146] und SZ-2 [Ruan, C. et al. (1987) A murine antiglycoprotein Ib complex monoclonal antibody, SZ 2, inhibits platelet aggregation induced by both ristocetin and collagen. Blood 69(2), 570-577; kommerziell erhältlich z. B. von Beckman Coulter Inc., Fullerton, USA: Produktnummer IM0409]. Sofern das verwendete GPIbα-Protein am C-Terminus mit einem oder mehreren Flag-Tags fusioniert ist, ist gleichermaßen ein anti-Flag-Antikörper geeignet [siehe z. B. US 5,011,912; kommerziell erhältlich z. B. von Sigma-Aldrich Chemie GmbH, Steinheim, Deutschland]. Zur quantitativen Bestimmung der Agglutinationsreaktion, die mit der Menge bzw. Aktivität des in der Probe vorhandenen VWF korreliert, kann z. B. die Lichtstreuung an den Partikelaggregaten über die Messung der Streulichintensität (Nephelometrie) oder über die Messung der Trübung des Mediums (Turbidimetrie) genutzt werden.

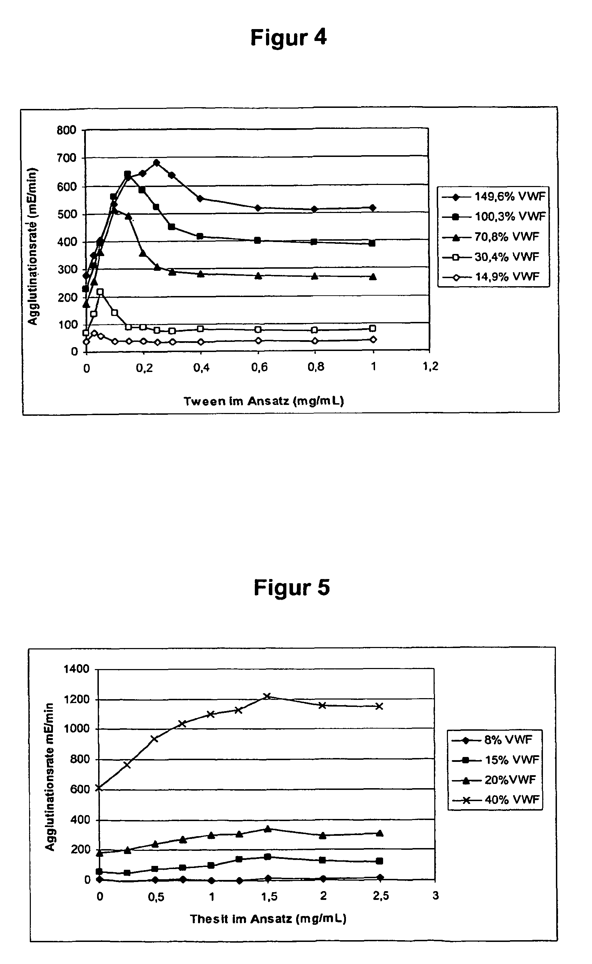

Bevorzugterweise wird die Bestimmung der VWF-Aktivität anhand der GPIbα-vermittelten Agglutination einer partikulären Festphase in Gegenwart eines Detergenzes durchgeführt, bevorzugterweise in Gegenwart eines Detergenzes aus der Gruppe Tween® 20, Thesit, Triton-Detergenz (z. B. Triton X-100® oder Triton X-405®) und Natriumdodecylsulfat (SDS). Es wurde gefunden, dass die Gegenwart eines Detergenzes die VWF-abhängige Agglutinationsrate der partikulären Festphase, insbesondere von Latexpartikeln, beeinflusst:
Bei verschiedenen VWF-Konzentrationen und bei steigenden Tween® 20-Konzentrationen wurde die Agglutinationsrate bestimmt (siehe Figur 4, Bestimmung analog Beispiel 5). Es wird deutlich, dass es bei relativ geringen Tween® 20-Konzentrationen (0,02-0,1 g/L) eine besonders ausgeprägte Verstärkung der Reaktion gibt, welche mit steigender Tween® 20-Konzentration wieder geringer wird und ein stabiles Niveau an Verstärkung erreicht. So ist z. B. bei 149,6 -% VWF bei ca. 1 g/L Tween® 20 das stabile Niveau der Verstärkung erreicht, und die Verstärkung beträgt dann 85 % vom Ausgangswert ohne Tween® 20. Wenn Tween® 20 als Detergenz benutzt wird, bestehen folglich zwei Möglichkeiten. Zum einen kann Tween® 20 bei geringen Konzentrationen (0,02-0,1 g/L) eingestellt werden, um bestimmte VWF-Konzentrationen bis zu 20 % besonders stark zu verstärken. So erhält man z. B. bei 0,05 g/L Tween® 20 (im Testansatz) eine Verstärkung der Agglutinationsrate um 216 % bei 30,4 % VWF. Da niedrige VWF-Konzentrationen besonders schwierig zu bestimmen sind, ist hier eine Verstärkung der Reaktion sehr hilfreich. Zum anderen ist ein Meßsystem generell stabiler, wenn im Sättigungsbereich des Detergenzes für alle relevanten VWF-Konzentrationen gearbeitet wird. Diese wäre z. B. ab 1 g/L Tween® 20 der Fall. Diese Variante ist besser für einen Screening-Test für ein weites Spektrum an VWF-Konzentrationen geeignet. Tween® 20-Konzentrationen von 0,6-20,0 g/L, bevorzugt von 1 g/L, sind vorteilhaft, wenn die Agglutinationsreaktion bei der Bestimmung von VWF-Konzentrationen von mehr als 20 % verstärkt werden soll.

Andere Detergenzien, wie Thesit (synonym: Polydocanol, Schärer & Schläpfer Ltd., Rothrist, Schweiz), Triton-Detergenz (z. B. Triton X-100® oder Triton X-405®) und Natriumdodecylsulfat (SDS) zeigen einen stetigen konzentrationsabhängigen Anstieg der Verstärkung der Agglutinationsreaktion (siehe Figur 5, Bestimmung analog Beispiel 5: 45 µL Probe jedoch mit Thesit anstelle von Tween® 20).

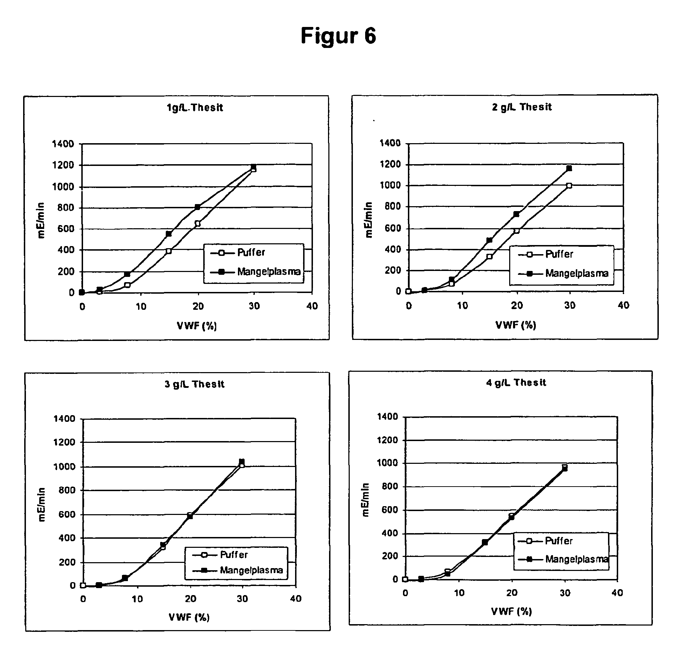

Um die VWF-Aktivität einer Plasmaprobe zu bestimmen, wird die Probe üblicherweise mit einem Puffer oder mit VWF-Mangelplasma vorverdünnt. Gleichermaßen wird mit Normalplasma (z. B. Standard Human Plasma) verfahren, wenn eine Kalibrationskurve erstellt werden soll. Es wurde festgestellt, dass es bei der Verdünnung mit VWF-Mangelplasma und bei einer üblichen Konzentration Detergenz (1 g/L im Testansatz ) zu einer unerwünschten Verstärkung der Agglutination der GPlba-Latexpartikel kommt, welche jedoch durch ausreichend Detergenz unterdrückt werden kann. Verdünnt man, wie in Figur 6 gezeigt, in einem Testansatz mit 45 µL Probe, die Probe (in diesem Fall Standard Human Plasma) mit VWF-Mangelplasma oder mit Puffer, so treten in Gegenwart von VWF-Mangelplasma höhere Agglutinationsraten bei einer bestimmten VWF-Konzentration in der Probe auf. Wird im Testsystem gänzlich auf Detergenz verzichtet und die Standard Human Plasma-Probe mit VWF-Mangelplasma verdünnt, so ergibt sich hingegen eine niedrigere Agglutinationsrate wie bei Verdünnung mit einem Puffer. Wird jedoch ausreichend Detergenz zugegeben (z. B. ≥ 3 g/L Thesit), so gleichen sich die Agglutinationsraten bei Verdünnung mit Puffer oder mit Mangelplasma an. Somit ist die Verdünnungsechtheit einer Plasmaprobe in einem Puffer gewährleistet. Für einen Test ist es äußerst vorteilhaft, wenn sowohl die Verdünnung eines Normalplasmas (als Kalibrator) wie auch eine eventuelle Verdünnung der Patientenprobe durch einen einfachen Puffer durchgeführt werden kann und nicht durch ein VWF-Mangelplasma durchgeführt werden muß. Ein Puffer ist preisgünstig, die Stabilität bei Lagerung besser zu gewährleisten und viele Testautomaten benutzen einen einzigen Puffer für viele Teste. Weiterhin ist VWF-Mangelplasma oft nicht in jedem Labor verfügbar. Bei der Messungen von VWF-Konzentraten werden besonders starke Verdünnungen durchgeführt, wobei eine gute Verdünnungsechtheit in Puffer ebenfalls sehr vorteilhaft ist.

Weiterhin bevorzugt ist die Bestimmung der VWF-Aktivität anhand der GPIbα-vermittelten Agglutination einer partikulären Festphase in Gegenwart von Polyvinylpyrrolidon (PVP), bevorzugterweise bei einer Polyvinylpyrrolidon-Konzentration von 0,1 % bis 1,0 % im Testansatz und/oder in Gegenwart von Dextran T500, bevorzugterweise bei einer Dextran T500-Konzentration von 0,1 % bis 3 % im Testansatz und/oder in Gegenwart von Alginate 500-600 cP, bevorzugterweise bei einer Alginate 500-600 cP-Konzentration von 0,01 % bis 0,2 % im Testansatz.

Bei einer anderen bevorzugten Ausführungsform des erfindungsgemäßen Verfahrens zur Bestimmung der VWF-Aktivität handelt es sich um ein kompetitives Testformat. Das Verfahren umfasst die Verwendung von Partikel-assoziiertem anti-GPIbα-Antikörper, bevorzugt VM16d, und von Partikel-assoziiertem VWF in einem Reaktionsansatz und die Zugabe von GPIbα-Protein. In Gegenwart des GPIbα-Proteins und in Abwesenheit von Ristocetin, Botrocetin oder einer äquivalenten Substanz agglutinieren die Partikel. Die Zugabe einer VWF-enthaltenden Probe hemmt diese Agglutinationsreaktion. Die Hemmung der Agglutinationsreaktion korreliert mit der Menge bzw. Aktivität des in der Probe vorhandenen VWF.

Die vorliegende Erfindung betrifft weiterhin ein Testkit zur Verwendung in dem erfindungsgemäßen Verfahren, wobei das Testkit mindestens ein Reagenz enthält, das GPIbα-Protein enthält, welches eine Aminosäuresequenz umfasst, die verglichen mit der Wildtypsequenz des humanen GPIbα-Proteins (SEQ ID NO:1) mindestens die Aminosäurereste 1-268 enthält und an den Positionen 233 und 239 jeweils eine Aminosäuresubstitution Xaa aufweist und das mit einer partikulären Festphase assoziiert ist. Bevorzugterweise enthält das Testkit ein Reagenz, das Latexpartikel-assoziiertes GPIbα-Protein enthält. Bevorzugterweise ist das GPIbα-Protein über einen Antikörper an die partikuläre Festphase assoziiert. Das Reagenz kann in flüssiger oder lyophilisierter Form bereitgestellt werden. Für den Fall, dass das Reagenz als Lyophilisat vorliegt, kann das Testkit zusätzlich ein zur Suspendierung des Lyophilisat erforderliches Lösemittel enthalten, wie z. B. destilliertes Wasser oder einen geeigneten Puffer.

### Figurenbeschreibung

**Figur 1**
   Der Aufbau der hier angewendeten GPIbα-(aa1-285,G233V/M239V)- bzw. Wildtyp-GPIbα-ELISA Testsysteme zur Bestimmung von VWF ist in Beispiel 4 angegeben. Es wurden 4 verschiedene Verdünnungen des VWF/Faktor VIII-Konzentrats Haemate® als Probe eingesetzt. In Abwesenheit von Ristocetin (0 mg/mL) erfolgt bei Verwendung des mutanten GPIbα-(aa1-285,G233V/M239V) eine starke und konzentrationsabhängige Bindung von VWF (Abbildung oben). Durch Zugabe von Ristocetin ist diese Bindung geringfügig steigerbar, was wahrscheinlich auf eine unspezifische Bindung zurückzuführen ist, die auch ohne GPIbα stattfindet. Hingegen ist eine ähnlich starke VWF-Bindung beim wildtypischen GPIbα nur durch Zugabe von 1,2 mg/mL Ristocetin zu erreichen. Ohne Ristocetinzugabe findet bei Verwendung von wildtypischem GPIbα überhaupt keine Bindung statt (Abbildung unten).
**Figur 2**
   Kalibrationskurve zur Bestimmung der VWF-Aktivität (10 % - 200 % VWF-Aktivität) in humanen Citratplasmaproben mittels eines GPIba-Latexpartikel-Agglutinationstests ohne Ristocetin (siehe Beispiel 5). Darstellung der Reaktionsgeschwindigkeit der Agglutination der Partikel in Abhängigkeit von der VWF-Aktivität.
**Figur 3**
   Kalibrationskurve zur Bestimmung niedriger VWF-Aktivitäten (≥ 3 % VWF-Aktivität) in humanen Citratplasmaproben mittels eines GPIbα-Latexpartikel-Agglutinationstests ohne Ristocetin (siehe Beispiel 5, 45 µL Probe). Darstellung der Reaktionsgeschwindigkeit der Agglutination der Partikel in Abhängigkeit von der VWF-Aktivität.
**Figur 4**
   GPIbα-Latexpartikel-Agglutinationstests ohne Ristocetin in Proben mit unterschiedlicher VWF-Konzentration (14,9 % bis 149,6 %) und in Gegenwart unterschiedlicher Tween® 20-Konzentrationen im Testansatz (siehe Beispiel 5, Variation der Tween® 20-Konzentration, 1-5 µL Probe).
**Figur 5**
   GPIbα-Latexpartikel-Agglutinationstests ohne Ristocetin in Proben mit unterschiedlicher VWF-Konzentration (8 % bis 40 %) und in Gegenwart unterschiedlicher Thesit-Konzentrationen im Testansatz (siehe Beispiel 5, jedoch Thesit anstelle von Tween® 20, 45 µL Probe).
**Figur 6**
   GPIbα-Latexpartikel-Agglutinationstests ohne Ristocetin in Proben mit unterschiedlicher VWF-Konzentration, die mit VWF-Mangelplasma bzw. mit Puffer vorverdünnt wurden, und in Gegenwart unterschiedlicher Thesit-Konzentrationen im Testansatz (siehe Beispiel 5, jedoch Thesit anstelle von Tween® 20, 45 µL Probe).

Die im Folgenden beschriebenen Beispiele dienen der exemplarischen Beleuchtung einzelner Aspekte dieser Erfindung und sind nicht als Einschränkung zu verstehen.

### Beispiele

### Beispiel 1: Klonierung des-GPIbα-(G233V/M239V)-(3xFlag)-(6xHis)-Konstrukts

Der Expressionsvektor pIRES neo2 (BD Biosciences, Clontech, Art.-Nr.: 6938-1) wurde so modifiziert, dass er zwischen der EcoRI- und der Notl-Restriktionsschnittstelle ein 3×Flag-Tag und ein 6xHis-Tag enthielt. Stromaufwärts (5') der Tag-Sequenzen wurde ein für das Signalpeptid (SEQ ID NO: 2) und die Aminosäuren 1-285 des humanen GPIbα-Rezeptors (SEQ ID NO: 1) codierendes Fragment, das an den für die Aminosäurepositionen 233 und 239 codierenden Stellen jeweils ein für Valin codierendes Codon enthielt, inseriert.

### Beispiel 2: Expression des rekombinanten GPIbα-(G233V/M239V)-(3×Flag)-(6×His)-Fusionsproteins in HEK-Zellen

HEK 293 Zellen (humane embryonale Nierenzellen; ATCC-Number: CRL-1573; Cell Lines Service CLS, Heidelberg) wurden mit dem in Beispiel 1 beschriebenen Konstrukt transformiert.

Die Zellen werden kultiviert in:
DMEM (Art.-Nr.:31966-021, Invitrogen)
+ 10% FBS Origin EU ( Art.-Nr.:10270-106, Invitrogen), das hitzeinaktiviert wurde oder aber in 10% FBS Origin USA ( Art.-Nr.:A15-003, Lot-No.:A01123-678, PAA)
+ 1% Antibiotic-Antimycotic-Solution(100x) (Art.-Nr.:P11-002, PAA)
+ 0,1% Gentamycin-Solution 50mg/ml ( Art.-Nr.:P11-005, PAA)
+ 500µg/ml Geneticin (G418)-Solution 50mg/ml active Geneticin (Art.-Nr.:10131-027, Invitrogen)

Für die Expression (Produktion):
OPTIPRO-SFM (Art.-Nr.:12309-019, Invitrogen)
+ 0,5% Antibiotic-Antimycotic-Solution(100x) (Art.-Nr.:P11-002, PAA)
+ 0,05% Gentamycin-Solution 50mg/ml ( Art.-Nr.:P11-005, PAA)
+ 2% Glutamax I (100X) (Art.-Nr.: 35050-038, Invitrogen)
und kein Geneticin

Der Ablauf ist wie folgt:
1) Auftauen der Zellen ( 1 Kryo mit 5-10x 10e6 Zellen) und Kultivierung in T175 in serumhaltigem Medium für 96h
2) Splitten auf 4x T175 und Kultivierung für 72-96h
3) Splitten auf 25x T175 und Kultivierung für 72-96h
4) Eine T175 splitten und als Reserve weiter kultivieren. Die restlichen 24x T175 splitten auf 3x CellStack Corning (je CellStack 10 Ebenen mit insgesamt 6360cm2) und für 72h kultivieren
5) Medium entfernen Monolayer mit DMEM ohne FBS 1-2x waschen und serumfreies OPTIPRO-SFM (1,8 Liter je CellStack) zugeben und für 96h kultivieren
6) Ernte des Mediums. Überstand abzentrifugieren und Pellet der abgelösten Zellen wiederaufnehmen und zurück in den CellStack mit frischem OPTIPRO-SFM geben und für 96h kultivieren
7) wie unter 6)
8) Finale Ernte des Mediums und Beendung der Kultur

### Beispiel 3: Affinitätschromatographische Isolierung des rekombinanten GPIbα-(G233V/M239V)-(3×Flag)-(6×His)-Fusionsproteins

Das nach Beispiel 2 gewonnene GPIbα-(G233V/M239V)-(3xFlag)-(6xHis) enthaltende Medium wird durch Zentrifugation (35 min, 10.000 UpM, Beckman J2-21, Beckman Coulter GmbH, Deutschland) von noch vorhanden Zellen oder Zellbruchstücken befreit. Der so erhaltene zellfreie Überstand wird mittels Tangentialultrafiltration unter Verwendung einer Ultraflitrationskassette mit einer Trenngrenze von 10 kDa (PES 10, Schleicher & Schüll, Deutschland) auf 1/10 des Ausgangsvolumens ankonzentriert.

Die Reinigung erfolgt mittels Affinitätschromatographie unter Verwendung einer Ni²⁺-Sepharose (His Prep FF16/10, GE Healthcare, Schweden) nach Angaben des Herstellers. Zur Bindung des GPIbα-(G233V/M239V)-(3×Flag)-(6×His) werden dem konzentrierten Überstand 500 mmol NaCl, 20 mmol Na₂HPO₄ und 5 mM Imidazol zugegeben und durch -Zugabe von 5 M HCl der pH-Wert auf pH 7,4 eingestellt. Nichtgebundene Komponenten werden durch Spülen der Säule mit einem Puffer aus 500 mmol NaCl, 20 mmol Na₂HPO₄, 5 mM Imidazol, pH 7,4 abgewaschen. Die Eluation des gebundenen GPIbα-(G233V/M239V)-(3×Flag)-(6xHis) erfolgt mit einem Puffer aus 20 mmol Na₂HPO₄, 500 mmol NaCl, 500 mMol Imidazol, pH 7,4. Das so erhaltene Eluat wird in einer Ultrafiltrationsrührzelle mit einer Ultrafiltrationsmembran, Trenngrenze 10 kDa (OMEGA 10 K, Pall Life Sciences, USA) auf 1/10 des Ausgangsvolumens ankonzentriert. Die weitere Reinigung und Abtrennung von Kontaminationen erfolgt mittels Gelfitration unter Verwendung einer Chromatographiesäule Superdex 200 prep grade 35/600 (GE Healthcare, Schweden) nach Angaben des Herstellers. Die Chromatographie erfolgt mit einer Flussrate von 5,0 ml/min unter Verwendung eines Puffers aus 0,048 mol/L Na₂HPO₄, 0,02 mol/L KH₂PO₄, 0,145 mol/L NaCl, 0,015 mol/L NaN₃, pH 7,2. Nach Auftragen der Probe eluiert GPIbα-(G233V/M239V)-(3×Flag)-(6×His) in einem Peak nach einem Eluationsvolumen von ca. 300 ml von der eingesetzten Chromatographiesäule.

### Beispiel 4 (nicht Teil der Erfindung): Verfahren zur Bestimmung der VWF-Aktivität in einem Anti-FLAG/GPIbα-ELISA ohne Ristocetin

Es wurden ELISA-Platten benutzt, die bereits mit einem Antikörper gegen das Flag-Tag beschichtet sind (Sigma, Saint Louis, USA, ANTI-FLAG HS, M2 coated 96-well Plates (clear), Product Number P 2983). In jede Vertiefung der ELISA-Platte wurden 100 µL einer Phosphatpufferlösung gegeben, die rekombinantes wildtypisches GPIbα-(3×Flag)-(6×His)-Fusionsprotein (1:10 verdünnter Überstand des Kulturmediums nach Zellsedimentation) oder isoliertes rekombinantes GPIbα-(aa1-285,G233V/M239V)-(3×Flag)-(6×His)-Fusionsprotein (siehe Beispiel 3) in einer Konzentration von 2,4 µg/mL enthielt, und es wurde über Nacht bei 2-8 °C inkubiert. Nach 4-fachem Waschen mit Phosphatpuffer (+ 0,01 % Tween®) wurden 50 µL einer Verdünnung von Hämate® (ZLB Behring, Marburg, Deutschland) in Phosphatpuffer + 0,1 % Rinderserumalbumin sowie 50 µL einer Ristocetin-Verdünnung in Phosphatpuffer + 0,1 % Rinderserumalbumin zugegeben. Danach erfolgte eine Inkubation für 1 Stunde bei Raumtemperatur. Nach 4-fachem Waschen wie oben wurden 100 µL eines horse raddish Peroxidase-gekoppelten anti-VWF-Antikörpers (Rabbit Anti-Human VWF/HRP, DAKO, Ref. P0226) zugegeben, 1 Stunde bei Raumtemperatur inkubiert und danach wie oben gewaschen. Daraufhin wurden 100 µL Tetramethylbenzidin-Lösung als Substrat (TMB Substrat, Dade Behring Marburg GmbH, Marburg, Deutschland, Ref. 450684) zugegeben, 4 Minuten bei Raumtemperatur inkubiert. Durch Zugabe von 100 µL 0,5 N Schwefelsäure wurde die Reaktion abgestoppt. Danach erfolgte die Messung der Extinktion bei 405 nm in einem Spektralphotometer.

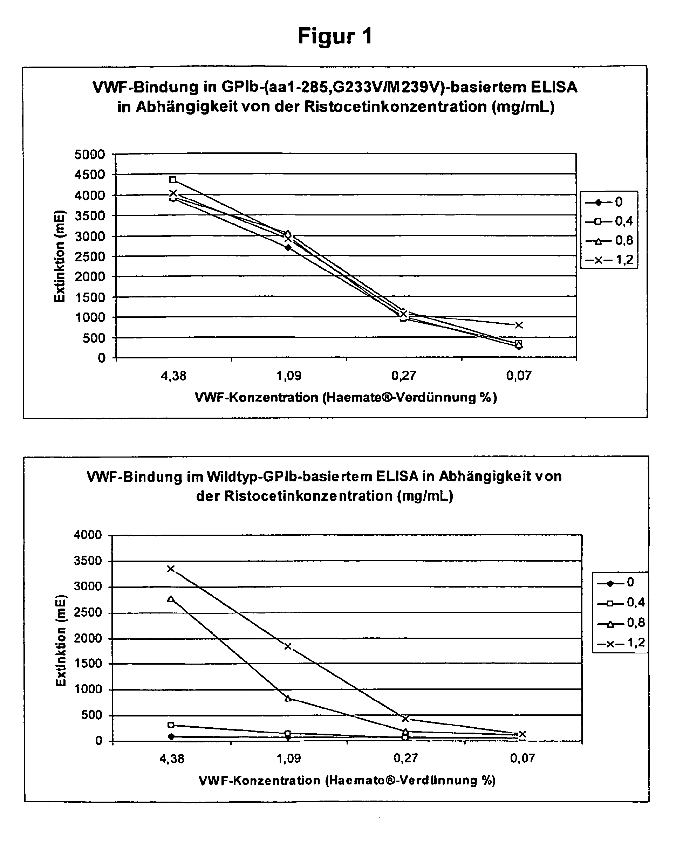

Bei Abwesenheit von Ristocetin erfolgte bei Verwendung des gemäß den Beispielen 1-3 hergestellten mutanten GPIbα eine starke und konzentrationsabhängige Bindung von VWF. Durch Zugabe von Ristocetin wurde diese Bindung geringfügig gesteigert, was wahrscheinlich auf eine unspezifische Bindung zurückzuführen ist, die auch ohne GPIbα stattfindet. Hingegen ist eine ähnlich starke VWF-Bindung beim wildtypischen GPIbα nur durch Zugabe von 1,2 mg/mL Ristocetin zu erreichen. Ohne Ristocetinzugabe findet bei Verwendung von wildtypischem GPIbα überhaupt keine Bindung statt (Figur 1).

### Beispiel 5: Erfindungsgemäßes Verfahren zur Bestimmung der VWF-Aktivität in einem GPIbα-Latexpartikel-Agglutinationstest ohne Ristocetin

Es wurden drei Reagenzien vorbereitet:
Reagenz 1:
   Der monoklonale anti-GPIbα-Antikörper VM16d (Sanbio B.V., Uden, Niederlande: Produktnummer MON 11-46) wurde auf der Oberfläche von Latexpartikeln gebunden, und die Partikel in einem Puffer zur Langzeitlagerung resuspendiert (0,6 g/L TRIS, 1,1 % Leucin, 12,5 % Saccharose, 0,05 % HSA, 6,25 mg/L Gentamycin und 0,625 mg/L Amphotericin, pH 8.25, Latexkonzentration 0,22 %).
Reagenz 2:
   VWF-Mangelplasma
Reagenz 3:
   Die folgenden Komponenten wurden gemischt und ergaben ein Gesamtvolumen von 30 mL Reagenz 3:
      2,5 mL einer 7,5 %igen Lösung von Polyvinylpyrrolidon (PVP) (Fluka, Deutschland) in Phosphatpuffer (pH 7,1),
      12,9 mL (0,625 mg/mL) Heterophilic Blocking Reagent 1 (Scantibodies Laboratory Inc, Santee, CA 92071, USA) in Tris-Puffer (pH 7,1),
      3,4 mL einer Lösung mit 21 g/L Tween® 20 (Sigma, St. Louis, Missouri, USA) in Phosphatpuffer (pH 7,2),
      0,086 mL einer Lösung von 5,59 mg/mL rekombinantem GPIbα-(G233V/M239V)-(3×Flag)-(6×His) (siehe Beispiel 3) in Phosphatpuffer (pH 7,1), und
      11,2 mL Phosphatpuffer (pH 7,1).

Die Testdurchführung erfolgte an dem automatischem Gerinnungsanalysengerät BCS® (Behring Coagulation System, Dade Behring Marburg GmbH, Marburg, Deutschland). Es wurden 15 µL einer humanen Citratplasma-Probe, 30 µL Reagenz 2 und 70 µL Reagenz 3 gemischt. Der Start der Reaktion erfolgte durch Zugabe und Mischung mit 40 µL Reagenz 1. Die Extinktion der Reaktionsmischung wurde kontinuierlich bei 575 nm gemessen. Die Geschwindigkeit der Agglutination der Latexpartikel erfolgte in Abhängigkeit von der Aktivität des VWF in der Probe.

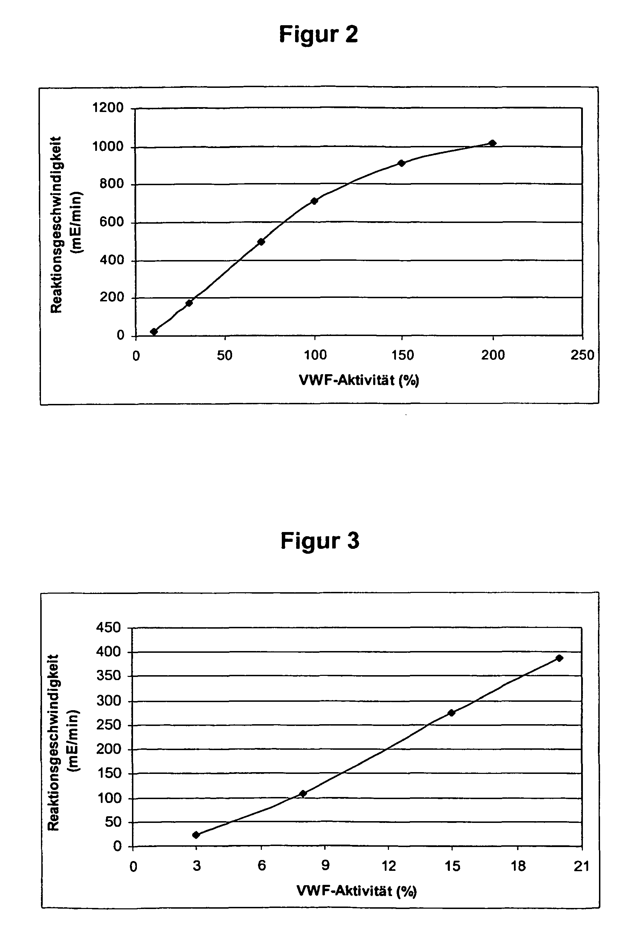

Zur Berechnung der VWF-Aktivität wurde eine Kalibrationskurve unter Benutzung von Standard Human Plasma (Dade Behring Marburg GmbH, Marburg, Deutschland) erstellt. Als VWF-Sollwert wurde der deklarierte Ristocetin-Kofaktor-Wert für das BCS®-System verwendet. Durch Erhöhung des Volumens von Standard Human Plasma bei gleichzeitiger Reduktion der entsprechende Menge an Reagenz 2 wurden VWF-Aktivitäten bis zu 200 % erzeugt. Durch Verdünnung des Standard Human Plasmas mit Reagenz 2 wurden niedrigere VWF-Aktivitäten erzeugt. Eine typische Kalibrationskurve ist in Figur 2 dargestellt. Die VWF-Aktivität einer Probe kann mit Hilfe der ermittelten Reaktionsgeschwindigkeit an der Kalibrationskurve abgelesen werden.

In einem Testansatz zur Messung besonders niedriger VWF-Aktivitäten in einer Probe wurden anstelle von 15 bzw. 30 µL Probe (siehe oben) 45 µL Probe eingesetzt. Bei der Kalibration wurde Standard Human Plasma mit Reagenz 2 so verdünnt, dass niedrige VWF-Aktivitäten von bis zu 3 % erzeugt wurden (siehe Figur 3). Dieser Testansatz ermöglicht die genaue Bestimmung extrem niedriger VWF-Aktivitäten, was besonders wichtig bei der Bildung von VWF-Aktivität/Antigen-Ratios ist, um Subtypen des Von Willebrand-Syndroms zu klassifizieren. Durch ein Erhöhung der PVP-Konzentration auf 0,35 % im Ansatz und des Probenvolumens auf 60 µL kann die VWF-abhängige Agglutination zusätzlich verstärkt werden und es kann noch eine Differenzierung von 1 % VWF und 0 % VWF erfolgen. Somit kann mit großer Sicherheit eine Typ 3-Von Willebrand Erkrankung (0 % VWF) von extrem starken Defizienzen anderer Typen (z. B. 1-5 %) unterschieden werden.

## Patentansprüche

1. Verfahren zur Bestimmung der von Willebrand Faktor (VWF)-Aktivität in einer Probe, wobei die Probe mit isoliertem GPIbα-Protein zu einem Testansatz vermischt wird, **dadurch gekennzeichnet, dass**
a) isoliertes GPIbα-Protein verwendet wird, das eine Aminosäuresequenz umfasst, die verglichen mit der Wildtypsequenz des humanen GPIbα-Proteins (SEQ ID NO:1) mindestens die Aminosäurereste 1-268 enthält und an den Positionen 233 und 239 jeweils eine Aminosäuresubstitution Xaa aufweist und dass
b) dem Testansatz kein Ristocetin und kein Botrocetin hinzugefügt wird und dass
c) das verwendete GPIbα-Protein mit einer partikulären Festphase assoziiert ist.

2. Verfahren nach Anspruch 1 **dadurch gekennzeichnet, dass** dem Testansatz keine Ristocetin- oder Botrocetin-äquivalente Substanz hinzugefügt wird

3. Verfahren nach einem der vorhergehenden Ansprüche **dadurch gekennzeichnet, dass** die Aminosäuresubstitution Xaa an Position 233 und/oder Position 239 des verwendeten GPIbα-Proteins aus einem Valin- oder einem Serin-Rest besteht.

4. Verfahren nach einem der vorhergehenden Ansprüche **dadurch gekennzeichnet, dass** das verwendete GPIbα-Protein rekombinant oder synthetisch hergestellt ist.

5. Verfahren nach einem der vorhergehenden Ansprüche **dadurch gekennzeichnet, dass** das verwendete GPIbα-Protein am N-Terminus mit der homologen, humanen GPIbα-Signalsequenz (SEQ ID NO: 2) oder einer heterologen Signalsequenz fusioniert ist.

6. Verfahren nach einem der vorhergehenden Ansprüche **dadurch gekennzeichnet, dass** das verwendete GPIbα-Protein am C-Terminus mit einem oder mehreren Affinitätstags fusioniert ist.

7. Verfahren nach Anspruch 6 **dadurch gekennzeichnet, dass** das verwendete GPIbα-Protein am C-Terminus mit einem oder mehreren Affinitätstags aus der Gruppe His-Tag, Flag-Tag, Arg-Tag, c-Myc-Tag und Strep-Tag fusioniert ist.

8. Verfahren nach einem der vorhergehenden Ansprüche **dadurch gekennzeichnet, dass** das verwendete GPIbα-Protein über einen Antikörper mit der partikulären Festphase assoziiert ist.

9. Verfahren nach Anspruch 8 **dadurch gekennzeichnet, dass** das verwendete GPIbα-Protein über einen anti-GPIbα-Antikörper mit der partikulären Festphase assoziiert ist.

10. Verfahren nach Anspruch 8 **dadurch gekennzeichnet, dass** das GPIbα-Protein über einen anti-Affinitäts-Tag-Antikörper mit der partikulären Festphase assoziiert ist.

11. Verfahren nach einem der vorhergehenden Ansprüche **dadurch gekennzeichnet, dass** die partikuläre Festphase aus Latex besteht.

12. Verfahren nach einem der vorhergehenden Ansprüche **dadurch gekennzeichnet, dass** die VWF-Aktivität anhand der GPIbα-vermittelten Agglutination der partikulären Festphase bestimmt wird.

13. Verfahren nach Anspruch 12 **dadurch gekennzeichnet, dass** das verwendete GPIbα-Protein über einen monoklonalen anti-GPIbα-Antikörper aus der Gruppe VM16d und SZ 2 mit der partikulären Festphase assoziiert ist.

14. Verfahren nach Anspruch 12 **dadurch gekennzeichnet, dass** das verwendete GPIbα-Protein am C-Terminus mit einem oder mehreren Flag-Tags fusioniert ist und über einen anti-Flag-Antikörper mit der partikulären Festphase assoziiert ist.

15. Verfahren nach einem der Ansprüche 12 bis 14 **dadurch gekennzeichnet, dass** der Testansatz ein Detergenz, bevorzugt ein Detergenz aus der Gruppe Tween® 20, Thesit, Triton-Detergenz und Natriumdodecylsulfat enthält.

16. Verfahren nach Anspruch 15 **dadurch gekennzeichnet, dass** der Testansatz Tween® 20 in einer Konzentration von 0,02 g/L bis 20 g/L enthält.

17. Verfahren nach einem der Ansprüche 1 bis 16 **dadurch gekennzeichnet, dass** die Probe eine biologische Flüssigkeit humanen oder tierischen Ursprungs ist.

18. Verfahren nach einem der Ansprüche 1 bis 16 **dadurch gekennzeichnet, dass** die Probe ein Reaktionsgemisch ist, bestehend aus einer biologischen Flüssigkeit, welcher isolierter VWF als Substrat zugesetzt ist.

19. Testkit zur Durchführung eines Verfahrens gemäß Anspruch 1 **dadurch gekennzeichnet, dass** es ein Reagenz enthält, das GPIbα-Protein enthält, welches eine Aminosäuresequenz umfasst, die verglichen mit der Wildtypsequenz des humanen GPIbα-Proteins (SEQ ID NO:1) mindestens die Aminosäurereste 1-268 enthält und an den Positionen 233 und 239 jeweils eine Aminosäuresubstitution Xaa aufweist und das mit einer partikulären Festphase assoziiert ist.

## Claims

1. A method for determining von Willebrand factor (VWF) activity in a sample, wherein said sample is mixed with isolated GPIbα protein to give an assay mix, which method comprises
a) using isolated GPIbα protein comprising an amino acid sequence which, when compared to the wild-type sequence of human GPIbα protein (SEQ ID NO: 1), includes at least the amino acid residues 1-268 and has in each case one amino acid substitution Xaa in positions 233 and 239, and
b) adding to the assay mix neither ristocetin nor botrocetin, and
c) associating the GPIbα protein used with a particulate solid phase.

2. The method as claimed in claim 1, wherein no ristocetin- or botrocetin-equivalent substance is added to the assay mix.

3. The method as claimed in either of the preceding claims, wherein the amino acid substitution Xaa in position 233 and/or position 239 of the GPIbα protein used comprises a valine or a serine residue.

4. The method as claimed in any of the preceding claims, wherein the GPIbα protein used is prepared recombinantly or synthetically.

5. The method as claimed in any of the preceding claims, wherein the GPIbα protein used is fused at the N terminus to the homologous, human GPIbα signal sequence (SEQ ID NO: 2) or a heterologous signal sequence.

6. The method as claimed in any of the preceding claims, wherein the GPIbα protein used is fused at the C terminus to one or more affinity tags.

7. The method as claimed in claim 6, wherein the GPIbα protein used is fused at the C terminus to one or more affinity tags from the group consisting of His tag, Flag tag, Arg tag, c-Myc tag and Strep tag.

8. The method as claimed in any of the preceding claims, wherein the GPIbα protein used is associated with the particulate solid phase via an antibody.

9. The method as claimed in claim 8, wherein the GPIbα protein used is associated with the particulate solid phase via an anti-GPIbα antibody.

10. The method as claimed in claim 8, wherein the GPIbα protein is associated with the particulate solid phase via an anti-affinity tag antibody.

11. The method as claimed in any of the preceding claims, wherein the particulate solid phase comprises latex.

12. The method as claimed in any of the preceding claims, wherein the VWF activity is determined on the basis of GPIbα-mediated agglutination of the particulate solid phase.

13. The method as claimed in claim 12, wherein the GPIbα protein used is associated with the particulate solid phase via a monoclonal anti-GPIbα antibody from the group consisting of VM16d and SZ 2.

14. The method as claimed in claim 12, wherein the GPIbα protein used is fused at the C terminus to one or more Flag tags and is associated with the particulate solid phase via an anti-Flag antibody.

15. The method as claimed in any of claims 12 to 14, wherein the assay mix includes a detergent, preferably a detergent from the group consisting of Tween® 20, Thesit, Triton detergent and sodium dodecyl sulfate.

16. The method as claimed in claim 15, wherein the assay mix contains Tween® 20 in a concentration of from 0.02 g/l to 20 g/l.

17. The method as claimed in any of claims 1 to 16, wherein the sample is a biological fluid of human or animal origin.

18. The method as claimed in any of claims 1 to 16, wherein the sample is a reaction mixture comprising a biological fluid to which isolated VWF has been added as substrate.

19. A test kit for carrying out a method as claimed in claim 1, which comprises a reagent containing GPIbα protein which comprises an amino acid sequence which, when compared to the wild-type sequence of human GPIbα protein (SEQ ID NO: 1), includes at least the amino acid residues 1-268 and has in each case one amino acid substitution Xaa in positions 233 and 239, and which reagent is associated with a particulate solid phase.

## Revendications

1. Procédé de détermination du facteur de von Willebrand ( VWF ) dans un échantillon, en mélangeant l'échantillon à de la protéine GPIbα isolée en une préparation de test, **caractérisé en ce que**
a) on utilise de la protéine GPIbα isolée, qui comprend une séquence d'acide aminé, qui, par rapport à la séquence de type sauvage de la protéine GPIbα humaine ( identification de séquence N° 1 ), contient au moins les restes 1 à 268 d'acide aminé et a, en les positions 233 et 239, respectivement, une substitution Xaa d'acide aminé et **en ce que**
b) on n'ajoute pas de ristocétine, ni de botrocétine à la préparation de test et **en ce que**
c) on associe la protéine GPIbα utilisée à une phase solide particulaire.

2. Procédé suivant la revendication 1, **caractérisé en ce qu'**on n'ajoute pas de substance équivalente à la ristocétine ou à la botrocétine à la préparation de test.

3. Procédé suivant l'une des revendications précédentes, **caractérisé en ce que** la substitution Xaa d'acide aminé, en la position 233 et/ou en la position 239, de la protéine GPIbα utilisée consiste en un reste de valine ou en un reste de sérine.

4. Procédé suivant l'une des revendications précédentes, **caractérisé en ce que** la protéine GPIbα utilisée est préparée de manière recombinante ou synthétique.

5. Procédé suivant l'une des revendications précédentes, **caractérisé en ce que** la protéine GPIbα utilisée est fusionnée sur la terminaison N à la séquence signal GPIbα homologue humaine ( identification de séquence N° 2 ) ou à une séquence signal hétérologue.

6. Procédé suivant l'une des revendications précédentes, **caractérisé en ce que** la protéine GPIbα utilisée est fusionnée sur la terminaison C à un ou à plusieurs marqueurs d'affinité.

7. Procédé suivant la revendication 6, **caractérisé en ce que** la protéine GPIbα est fusionnée sur la terminaison C à un ou à plusieurs marqueurs d'affinité choisis dans le groupe His-Tag, Flag-Tag, Arg-Tag, c-Myc-Tag et Strep-Tag.

8. Procédé suivant l'une des revendications précédentes, **caractérisé en ce que** la protéine GPIbα utilisée est associée à la phase solide particulaire par un anticorps.

9. Procédé suivant la revendication 8, **caractérisé en ce que** la protéine GPIbα utilisée est associée à la phase solide particulaire par un anticorps anti-GPIbα.

10. Procédé suivant la revendication 8, **caractérisé en ce que** la protéine GPIbα est associée à la phase solide particulaire par un anticorps d'antimarqueur d'affinité.

11. Procédé suivant l'une des revendications précédentes, **caractérisé en ce que** la phase solide particulaire est en latex.

12. Procédé suivant l'une des revendications précédentes, **caractérisé en ce qu'**on détermine l'activité VWF à l'aide de l'agglutination de la phase solide particulaire procurée par la GPIbα.

13. Procédé suivant la revendication 12, **caractérisé en ce que** la protéine GPIbα utilisée est associée à la phase solide particulaire par un anticorps anti-GPIbα monoclonal choisi dans le groupe VM16d et SZ 2.

14. Procédé suivant la revendication 12, **caractérisé en ce que** la protéine GPIbα utilisée est fusionnée sur la terminaison C à un ou à plusieurs marqueurs d'affinité Flag et est associée à la phase solide particulaire par un anticorps anti-Flag.

15. Procédé suivant l'une des revendications 12 à 14, **caractérisé en ce que** la préparation de test contient un détergent, de préférence un détergent du groupe Tween 20 ( marque de fabrique ), Thesit, détergent Triton et dodécylsulfate de sodium.

16. Procédé suivant la revendication 15, **caractérisé en ce que** la préparation de test contient du Tween 20 ( marque de fabrique ) en une concentration de 0,02 g/L à 20 g/L.

17. Procédé suivant l'une des revendications 1 à 16, **caractérisé en ce que** l'échantillon est un liquide biologique d'origine humaine ou animale.

18. Procédé suivant l'une des revendications 1 à 16, **caractérisé en ce que** l'échantillon est un mélange réactionnel constitué d'un liquide biologique, auquel du VWF isolé est ajouté comme substrat.

19. Trousse de test pour effectuer un procédé suivant la revendication 1, **caractérisée en ce qu'**elle contient un réactif, qui contient de la protéine GPIbα, qui comprend une séquence d'acide aminé, qui, par rapport à la séquence de type sauvage de la protéine GPIbα humaine ( identification de séquence N° 1 ), contient au moins les restes 1 à 268 d'acide aminé et a, en les positions 233 et 239, respectivement, une substitution Xaa d'acide aminé et qui est associée à une phase solide particulaire.


### SEQUENCE LISTING

<110> Dade Behring Marburg GmbH
<120> Verfahren zur Bestimmung der von Willebrand Faktor-Aktivität in Abwesenheit von Ristocetin und zur Bestimmung der ADAMTS-13-Protease
<130> 2007/B005- Ma 1289
<150> DE 10 2007 031 708.7
   <151> 2007-07-06
<160> 3
<170> PatentIn version 3.3
<210> 1
   <211> 610
   <212> PRT
   <213> Homo sapiens
<400> 1
   His Pro Ile Cys Glu Val Ser Lys Val Ala Ser His Leu Glu Val Asn
   1               5                   10                  15
   Cys Asp Lys Arg Asn Leu Thr Ala Leu Pro Pro Asp Leu Pro Lys Asp
               20                  25                  30
   Thr Thr Ile Leu His Leu Ser Glu Asn Leu Leu Tyr Thr Phe Ser Leu
           35                  40                  45
   Ala Thr Leu Met Pro Tyr Thr Arg Leu Thr Gln Leu Asn Leu Asp Arg
       50                  55                  60
   Cys Glu Leu Thr Lys Leu Gln Val Asp Gly Thr Leu Pro Val Leu Gly
   65                  70                  75                  80
   Thr Leu Asp Leu Ser His Asn Gln Leu Gln Ser Leu Pro Leu Leu Gly
                   85                  90                  95
   Gln Thr Leu Pro Ala Leu Thr Val Leu Asp Val Ser Phe Asn Arg Leu
               100                 105                 110
   Thr Ser Leu Pro Leu Gly Ala Leu Arg Gly Leu Gly Glu Leu Gln Glu
           115                 120                 125
   Leu Tyr Leu Lys Gly Asn Glu Leu Lys Thr Leu Pro Pro Gly Leu Leu
       130                 135                 140
   Thr Pro Thr Pro Lys Leu Glu Lys Leu Ser Leu Ala Asn Asn Asn Leu
   145                 150                 155                 160
   Thr Glu Leu Pro Ala Gly Leu Leu Asn Gly Leu Glu Asn Leu Asp Thr
                   165                 170                 175
   Leu Leu Leu Gln Glu Asn Ser Leu Tyr Thr Ile Pro Lys Gly Phe Phe
               180                 185                 190
   Gly Ser His Leu Leu Pro Phe Ala Phe Leu His Gly Asn Pro Trp Leu
           195                 200                 205
   Cys Asn Cys Glu Ile Leu Tyr Phe Arg Arg Trp Leu Gln Asp Asn Ala
       210                 215                 220
   Glu Asn Val Tyr Val Trp Lys Gln Gly Val Asp Val Lys Ala Met Thr
   225                 230                 235                 240
   Ser Asn Val Ala Ser Val Gln Cys Asp Asn Ser Asp Lys Phe Pro Val
                   245                 250                 255
   Tyr Lys Tyr Pro Gly Lys Gly Cys Pro Thr Leu Gly Asp Glu Gly Asp
               260                 265                 270
   Thr Asp Leu Tyr Asp Tyr Tyr Pro Glu Glu Asp Thr Glu Gly Asp Lys
           275                 280                 285
   Val Arg Ala Thr Arg Thr Val Val Lys Phe Pro Thr Lys Ala His Thr
       290                 295                 300
   Thr Pro Trp Gly Leu Phe Tyr Ser Trp Ser Thr Ala Ser Leu Asp Ser
   305                 310                 315                 320
   Gln Met Pro Ser Ser Leu His Pro Thr Gln Glu Ser Thr Lys Glu Gln
                   325                 330                 335
   Thr Thr Phe Pro Pro Arg Trp Thr Pro Asn Phe Thr Leu His Met Glu
               340                 345                 350
   Ser Ile Thr Phe Ser Lys Thr Pro Lys Ser Thr Thr Glu Pro Thr Pro
           355                 360                 365
   Ser Pro Thr Thr Ser Glu Pro Val Pro Glu Pro Ala Pro Asn Met Thr
       370                 375                 380
   Thr Leu Glu Pro Thr Pro Ser Pro Thr Thr Pro Glu Pro Thr Ser Glu
   385                 390                 395                 400
   Pro Ala Pro Ser Pro Thr Thr Pro Glu Pro Thr Pro Ile Pro Thr Ile
                   405                 410                 415
   Ala Thr Ser Pro Thr Ile Leu Val Ser Ala Thr Ser Leu Ile Thr Pro
               420                 425                 430
   Lys Ser Thr Phe Leu Thr Thr Thr Lys Pro Val Ser Leu Leu Glu Ser
           435                 440                 445
   Thr Lys Lys Thr Ile Pro Glu Leu Asp Gln Pro Pro Lys Leu Arg Gly
       450                 455                 460
   Val Leu Gln Gly His Leu Glu Ser Ser Arg Asn Asp Pro Phe Leu His
   465                 470                 475                 480
   Pro Asp Phe Cys Cys Leu Leu Pro Leu Gly Phe Tyr Val Leu Gly Leu
                   485                 490                 495
   Phe Trp Leu Leu Phe Ala Ser Val Val Leu Ile Leu Leu Leu Ser Trp
               500                 505                 510
   Val Gly His Val Lys Pro Gln Ala Leu Asp Ser Gly Gln Gly Ala Ala
           515                 520                 525
   Leu Thr Thr Ala Thr Gln Thr Thr His Leu Glu Leu Gln Arg Gly Arg
       530                 535                 540
   Gln Val Thr Val Pro Arg Ala Trp Leu Leu Phe Leu Arg Gly Ser Leu
   545                 550                 555                 560
   Pro Thr Phe Arg Ser Ser Leu Phe Leu Trp Val Arg Pro Asn Gly Arg
                   565                 570                 575
   Val Gly Pro Leu Val Ala Gly Arg Arg Pro Ser Ala Leu Ser Gln Gly
               580                 585                 590
   Arg Gly Gln Asp Leu Leu Ser Thr Val Ser Ile Arg Tyr Ser Gly His
           595                 600                 605
   Ser Leu
       610
<210> 2
   <211> 16
   <212> PRT
   <213> Homo sapiens
<400> 2
   Met Pro Leu Leu Leu Leu Leu Leu Leu Leu Pro Ser Pro Leu His Pro
   1               5                   10                  15
<210> 3
   <211> 41
   <212> PRT
   <213> Artificial
<220>
   <223> heterologous signal peptide
<400> 3
   Met Pro Leu Gln Leu Leu Leu Leu Leu Ile Leu Leu Gly Pro Gly Asn
   1               5                   10                  15
   Ser Leu Gln Leu Trp Asp Thr Trp Ala Asp Glu Ala Glu Lys Ala Leu
               20                  25                  30
   Gly Pro Leu Leu Ala Arg Asp Arg Arg
           35                  40